(19) Europäisches Patentamt
European Patent Office
Office européen des brevets

(11) EP 2 389 924 B1

(12) EUROPEAN PATENT SPECIFICATION

(45) Date of publication and mention
of the grant of the patent:
**27.08.2014 Bulletin 2014/35**

(51) Int Cl.:
*A61K 9/08* *(2006.01)*      *A61K 35/14* *(2006.01)*
*A61K 47/36* *(2006.01)*      *A61P 27/02* *(2006.01)*
*A61K 9/00* *(2006.01)*

(21) Application number: **09838603.0**

(22) Date of filing: **16.12.2009**

(86) International application number:
**PCT/CN2009/001475**

(87) International publication number:
**WO 2010/083636 (29.07.2010 Gazette 2010/30)**

(54) **AN EYEDROPS OF THE DEPROTEINIZED CALF BLOOD EXTRACT**

AUGENTROPFEN DES DEPROTEINISIERTEN KÄLBERBLUTEXTRAKTS

COLLYRE À BASE D'EXTRAIT DE SANG DE VEAU DÉPROTÉINÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.01.2009 CN 200910000893**

(43) Date of publication of application:
**30.11.2011 Bulletin 2011/48**

(73) Proprietor: **Shenyang Xingqi Pharmaceutical Co., Ltd.**
**Liaoning 110027 (CN)**

(72) Inventors:
• **LIU, Jidong**
**Liaoning 110027 (CN)**
• **AI, Licheng**
**Liaoning 110027 (CN)**
• **YANG, Yuchun**
**Liaoning 110027 (CN)**

(74) Representative: **Inspicos A/S**
**Kogle Allé 2**
**P.O. Box 45**
**2970 Hørsholm (DK)**

(56) References cited:
**CN-A- 1 486 701**     **CN-A- 1 586 499**
**CN-A- 1 895 275**

## EP 2 389 924 B1

**Description**

TECHNICAL FIELD

The present invention relates to an eye drops comprising a deproteinized calf blood extract.

BACKGROUND ART

Deproteinized calf blood extract is a biologics invented by German researcher Jaeger, which is a deproteinized blood extract having a molecular weight of less than 6000 prepared by a membrane filtration technology from the blood of calf that is younger than 6 months, healthy and well developed. Such deproteinized blood extract contains electrolytes, essential trace elements as well as nucleic acid derivatives, amino acids, oligosaccharides, low molecular polypeptides, glycolipids and intermediates of lipids metabolism and the like. The deproteinized calf blood extract is able to prompt the uptake and utilization of glucose and oxygen in cell mitochondria (independent of insulin) at the cell level, allow the increased ATP synthesis, elevated cell energy and the activation of various enzymes. Under the conditions of low blood oxygen and increased energy demand, it is a high effective cellular nutrient and cellular growth factor to rapidly improve anoxic state of tissue cells, improve microcirculation, activate proliferation of tissue cells, prompt repair of damaged tissue cells.

The deproteinized calf blood extract does not contain antigens, has a good tolerance, is safe and non-toxic, and exhibits good effects for clinic application as a drug for prompting cellular energy metabolism. Ophthalmic liquid preparations of the deproteinized calf blood extract clinically is mainly used for the treatment of traumatic, inflammatory and trophic pathology of cornea and conjunctiva, and xerophthalmia by improving the uptake and utilization of oxygen and glucose in tissue cells and improving local blood circulation and nutritional disorders, as well as prompting the transmigration and proliferation of fibroblasts and vascular endothelial cells and the like. Currently, the new drugs marketed for the treatment of such disorders include: recombinant bovine basic fibroblast growth factor, and recombinant human epidermal growth factor, which both are products of genetic engineering. Although they theoretically have an effect for prompting epidermal growth, their effects are far from the medium required practically in the growth of body surface, and they are still not comparable to the calf blood extract.

An ophthalmic ointment of the deproteinized calf blood extract has been marketed abroad already. For example, a product with a trade name of "Solcoseryl" manufactured in Switzerland is an ophthalmic ointment containing the deproteinized calf blood extract as the active ingredient, which has significantly therapeutic effects on the repair of damaged ophthalmic tissues. However, no ophthalmic preparations of the deproteinized calf blood extract, especially eye drops are clinically applied in China.

CN1895275 discloses a gel comprising 20-50 portions by weight of deproteinized calf blood extract, 0.5-1.2 portions of carbomer, 45-80 portions of water for injection, 0.2-0.5 portions of NaOH for adjusting pH and 0.05-0.1 portions of a bacteriostatic agent. The gel is used for curing corneal and conjunctival disease as well as xerophthalmia.

Although an injection of the deproteinized calf blood extract has been clinically applied in China, the inventors of the present invention has found that such injection containing Tween-80 is unsuitable to be used as ophthalmic preparation especially as eye drops because of the un-ideal stability. Hence, there is still a need for the person skilled in the art to develop an ophthalmic preparation of the deproteinized calf blood extract, especially an eye drops, which is suitable for use in clinic applications.

CONTENTS OF THE INVENTION

The object of the present invention is to provide an eye drops comprising a deproteinized calf blood extract suitable for clinic application. The inventors of the present invention have found that the stability of the deproteinized calf blood extract can be effectively increased by using a chitosan oligosaccharide. Based on this discovery, the present invention has been completed by the inventors.

The present invention provides an eye drops for ophthalmic application, which comprises a therapeutically effective amount of a deproteinized calf blood extract, a chitosan oligosaccharide and water.

In one embodiment of the eye drops of the present invention, based on the total amount of the eye drops, it comprises 10-30% (v/v) of the deproteinized calf blood extract, 0.01-1% (w/v) of the chitosan oligosaccharide, and water.

In one embodiment of the eye drops of the present invention, the chitosan oligosaccharide has a molecular weight of less than 5,000, preferably less than 3,000, preferably less than 2,000, and preferably less than 1,500.

In one embodiment of the eye drops of the present invention, it further comprises one or more bacteriostatic agents selected from phenethanol, phenoxyethanol, methyl p-hydroxybenzoate, ethyl p-hydroxybenzoate, propyl p-hydroxybenzoate, benzalkonium chloride, and trichloro-tert-butanol. In a further embodiment, the bacteriostatic agent is one or more agents selected from phenoxyethanol, ethyl p-hydroxybenzoate, benzalkonium chloride, and trichloro-tert-butanol. In a further embodiment, the bacteriostatic agent is the combination of phenoxyethanol and ethyl p-hydroxybenzoate. In a still further embodiment, the bacteriostatic agent presents in an amount of 0.005-1% (w/v) based on the total amount of the eye drops. Preferably, the bacteriostatic agent presents in an amount of 0.01-0.5% (w/v) based on the total amount of the eye drops. Preferably, the bacteriostatic agent presents in an amount of 0.1-0.5% (w/v) based on the total amount of the eye drops. Preferably, the bacteriostatic agent presents in an amount of 0.01-0.1% (w/v) based on the total amount of the eye drops. Preferably, the bacteriostatic agent presents in an amount of 0.01, 0.1 or 0.5% (w/v) based on the total amount of the eye drops.

In one embodiment of the eye drops of the present invention, it further comprises a pH regulator. In a further embodiment, the eye drops has a pH value of 6.0-9.0, preferably 6.0-8.5, preferably 6.0-8.0. In a still further embodiment, the pH regulator is selected from phosphates, acetates, borates and combinations thereof, such as the combination of sodium dihydrogen phosphate and disodium hydrogen phosphate, the combination of sodium acetate and acetic acid, the combination of sodium borate and boric acid and the like. The person skilled in the art can determine the amount of the above pH regulators depending on specific conditions.

In one embodiment of the eye drops of the present invention, it further comprises an osmotic regulator. Examples of the osmotic regulator include sodium chloride, boric acid, potassium nitrate, and glucose, etc., and the person skilled in the art can determine the amount of the osmotic regulators depending on specific conditions.

In one embodiment of the eye drops of the present invention, it further comprises a viscosity regulator, and such viscosity regulator is also understood as a thickening agent by the person skilled in the art. Examples of the viscosity regulator of the present invention include polyvinylpyrrolidone, sodium carboxymethyl cellulose, methyl cellulose, and polyvinyl alcohol, etc. The person skilled in the art can determine the amount of the viscosity regulator depending on specific conditions.

The various aspects of the present invention and benefits thereof are further illustrated as follows.

As for all documents cited herein, their full disclosures are incorporated into herein by reference, and if the meanings expressed in these documents are not in consistence with the present invention, the expressions of the present invention should be used. In addition, the terms and phrases used in the present invention have the general meanings well known by the person skilled in the art, and nevertheless, these terms and phrases are further explained and specified herein in detail. If the meanings of the mentioned terms and phrases are not in consistence with those well known in the art, the meanings defined herein should be used.

In the context of the present invention, the symbols "v/v" and "w/v" used for % respectively refer to the percentages of "volume/volume" (i.e., ml/100ml) and the percentage of "weight/volume" (i.e., g/100ml).

The deproteinized calf blood extract used in the present invention is obtained by taking blood from a calf aged less than 6 months, and subjecting the blood to processess such as deproteinization, virus inactivation, and cuting off by ultra-filtration. The deproteinized calf blood extract should meet the requirements of "Quality standard of the deproteinized calf blood extract" (which standard is one approved by the State Food and Drug Administration with the Number of YBH 05782007, and a approval date of December 29, 2007). The extract is prepared by deproteinizing, concentrating, and ultra-filtering the calf blood, and contains active ingredients such as amino acids, and small molecular peptides. The total solid content should not be less than 35 mg/ml.

The preparation and/or quality indices of the deproteinized calf blood extract of the present invention are further illustrated in detail as follows. The deproteinized calf blood extract as a raw material used in the eye drops of the present invention may be a deproteinized calf blood extract with a desired molecular weight obtained by using the blood as the raw material from the calf of cattle species in the northern region of China younger than 6 months with a quarantine certificate from Songnen Plain ranch of Helongjiang Province (which ranch is one of the three excellent quality ranches in the world), removing fibrins, concentrating and cutting off by ultra-filtration of membrane separation technology under decontamination conditions. After being subjected to inactivation of virus, the extract contains active ingredients such as amino acids, and low molecular weight peptides and can be used as a raw material in an ophthalmic gel and eye drops mainly for the treatment of traumatic, inflammatory and trophic disorders of cornea and conjunctiva. The specific process of preparation comprises: (1) deproteinizing the calf blood: adding an extracting agent into the thawed, defibrinized calf blood in the desired proportion under aseptic conditions, stirring for the prescribed time, and standing for a period of time at the prescribed conditions of temperature and humidity; (2) evaporating and concentrating: concentrating at a reduced pressure by using a rotary evaporator; and (3) cutting off by ultra-filtration: subjecting the concentrate with the active ingredients cut off to a membrane separation technology to obtain the deproteinized calf blood extract. The ophthalmic preparation formulated from the deproteinized calf blood extract should be controlled to have a measured activity of not less than 4.0 $\mu$l $O_2$/mg.h, and the eye drops should contain polypeptides of 0.18mg - 0.28 mg per ml.

The chitosan oligosaccharide ($\beta$-1,4-linked-D-glucosamine, also called as chitin oligosaccharide, oligosaccharins) of the present invention refers to an oligosaccharide consists of 2-10 glucosamines linked by $\beta$-1,4-glucosidic bonds, which usually is obtained by degrading chitosan which is a product of de-acetylation of chitin (shells of shrimp and crab). Chitosan oligosaccharide can be obtained by the known methods, or available directly by commercial approaches, for example, from Dailian Glycobio Co., Ltd. Chitosan oligosaccharides with different molecular weights can be used depending on the demands. In one embodiment of the present invention, the chitosan oligosaccharide has a molecular weight of less than 5000. In a further embodiment of the present invention, the chitosan oligosaccharide has a molecular weight of less than 3000. In a still further embodiment of the present invention, the chitosan oligosaccharide has a molecular weight of less than 2000. In a still further embodiment of the present invention, the chitosan oligosaccharide has a molecular weight of less than 1500. In one embodiment of the eye drops of the present invention, the content of the chitosan oligosaccharide is 0.01-1% (w/v). In another embodiment of the eye drops of the present invention, the content of the chitosan oligosaccharide is 0.01-0.5% (w/v). In still another embodiment of the eye drops of the present invention, the content of the chitosan oligosaccharide is 0.05-0.2% (w/v). In further another embodiment of the eye drops of the present invention, the content of the chitosan oligosaccharide is 0.05 or 0.1 % (w/v).

The eye drops of the present invention is an aqueous composition, in which water is used as the medium or solvent or excipient or carrier of the composition. Although the percentage of water in the eye drops of the present invention relative to the total weight of eye drops, or the amount of water used for formulating the eye drops of the present invention is not specifically given, the person skilled in the art clearly know that as the medium or solvent or excipient or carrier of the eye drops, the amount of water is calculated by adding water to reach the total weight of the eye drops, which is a calculation method usually adopted in the formulation of a liquid preparation such as an eye drops or injection.

In the eye drops of the present invention, the bacteriostatic agent can be any combination of one or more bacteriostatic agents. In one embodiment, the bacteriostatic agent is the combination of phenoxyethanol and ethyl p-hydroxybenzoate; in a further embodiment, the bacteriostatic agent is the combination of phenoxyethanol and ethyl p-hydroxybenzoate in any ratios; in a still further embodiment, the bacteriostatic agent is a combination of phenoxyethanol and ethyl p-hydroxybenzoate in a weight ratio of 50:1. In one embodiment, the bacteriostatic agent is the combination of phenoxyethanol and benzalkonium chloride; in a further embodiment, the bacteriostatic agent is the combination of phenoxyethanol and benzalkonium chloride in any ratios; in a still further embodiment, the bacteriostatic agent is the combination of phenoxyethanol and benzalkonium chloride in a weight ratio of 50:1. In one embodiment, the bacteriostatic agent is the combination of trichloro-tert-butanol and benzalkonium chloride; in a further embodiment, the bacteriostatic agent is the combination of trichloro-tert-butanol and benzalkonium chloride in any ratios; in a still further embodiment, the bacteriostatic agent is the combination of trichloro-tert-butanol and benzalkonium chloride in a weight ratio of 10:1. In one embodiment, the bacteriostatic agent is the combination of trichloro-tert-butanol and ethyl p-hydroxybenzoate; in a further embodiment, the bacteriostatic agent is the combination of trichloro-tert-butanol and ethyl p-hydroxybenzoate in any ratios; in a still further embodiment, the bacteriostatic agent is the combination of trichloro-tert-butanol and ethyl p-hydroxybenzoate in a weight ratio of 10:1.

The second aspect of the present invention can provide such eye drops that comprises the eye drops according to any embodiments of the first aspect of the present invention, and additionally a pharmaceutically acceptable excipient. For example, the eye drops of the first aspect of the present invention can be diluted with a suitable vehicle to form the ready-to-use eye drops of the second aspect of the present invention just before clinic application in order to achieve some effects suitable for clinic application. For example, when the environmental pH suitable for long-term storage of the eye drops of the first aspect of the present invention is not consistent with the physiological pH, a pH regulator in a suitable amount can be added to the eye drops of the first aspect of the present invention just before clinic application to obtain an eye drops of the second aspect of the present invention with a pH value adjusted to the physiologically acceptable pH.

The present invention further relates to an eye drops with a formula of any one of examples of the present invention.

The present invention further relates to an eye drops with the following formula:

| | |
|---|---|
| Deproteinized calf blood extract | 200 parts by weight, |
| Phenoxyethanol | 5 parts by weight, |
| Benzalkonium chloride | 0.1 parts by weight, |
| Chitosan oligosaccharide | 0.5 parts by weight, |
| Water for injection | q.s., to 1000 parts by weight. |

The present invention further relates to an eye drops with the following formula:

| | |
|---|---|
| Deproteinized calf blood extract | 200 parts by weight, |
| Phenoxyethanol | 5 parts by weight, |
| Ethyl p-hydroxybenzoate | 0.1 parts by weight, |
| Chitosan oligosaccharide | 0.5 parts by weight, |
| Water for injection | q.s., to 1000 parts by weight. |

The present invention further relates to an eye drops with the following formula:

| | |
|---|---|
| Deproteinized calf blood extract | 200 parts by weight, |
| Trichloro-tert-butanol | 1 parts by weight, |
| Benzalkonium chloride | 0.1 parts by weight, |
| Chitosan oligosaccharide | 1.0 part by weight, |
| Water for injection | q.s., to 1000 parts by weight. |

The present invention further relates to an eye drops with the following formula:

| | |
|---|---|
| Deproteinized calf blood extract | 200 parts by weight, |
| Trichloro-tert-butanol | 1.0 part by weight, |
| Ethyl p-hydroxybenzoate | 0.1 parts by weight, |
| Chitosan oligosaccharide | 1.0 part by weight, |
| Water for injection | q.s., to 1000 parts by weight. |

The third aspect of the present invention provides a use of the eye drops according to any one embodiment of the first aspect of the present invention or the eye drops according to any one embodiment of the second aspect of the present invention in the manufacture of a medicament for the treatment of ophthalmic diseases (for example, keratopathy such as corneal injury, pathological changes of conjunctiva, and xerophthalmia).

The fourth aspect of the present invention provides a method for the treatment and/or prophylaxis of ophthalmic diseases (for example, keratopathy such as corneal injury, pathological changes of conjunctiva, and xerophthalmia), comprising administering the eye drops according to any one embodiment of the first aspect of the present invention or the eye drops according to any one embodiment of the second aspect of the present invention in a therapeutically and/or prophylactically effective amount to a subject in need thereof.

As used herein, the term "subject" refers to but is not limited to mammals such as pigs, dogs, monkeys, cats, human, and birds such as chickens, and ducks, etc., preferably, the subject is human, especially a human suffering from or possibly suffering from the ophthalmic diseases (for example, keratopathy such as corneal injury, pathological changes of conjunctiva, and xerophthalmia) as mentioned herein.

The eye drops of the present invention can be prepared by the following process: weighing the chitosan oligosaccharide in the prescribed amounts (and, if desired, further comprising a bacteriostatic agent such as the combination of phenoxyethanol and ethyl p-hydroxybenzoate, a pH regulator, an osmotic regulator, and a viscosity regulator, etc.) under aseptic conditions, dissolving it with water for injection in a suitable amount, adding the deproteinized calf blood extract, stirring uniformly, adding water for injection close to the total amount. The solution is filtrated by 0.45 $\mu$m microporous filtration membrane, then added with water for injection to reach the total amount, and finally filtrated by 0.22 $\mu$m microporous membrane to remove bacteria, thereby obtaining the eye drops. The eye drops of the deproteinized calf blood extract prepared according to the present invention has a fixed source of the calf blood source, a stable quality, and an easily controllable process of production. The obtained ophthalmic liquid preparation of the deproteinized calf blood extract has clear and transparent appearance, free of bacteria, low irritation, low toxicity, and is a safe and effective ophthalmic preparation useful for clinic application.

The eye drops of the deproteinized calf blood extract of the present invention can promote oxygen availability of cells by enhancing the respiration ability of mitochondria and the synthesis of high energy phosphate, and maintain physiological functions of important enzymes in human body. Hence, it has functions of improving tissue nutrition, stimulating cell reproduction and accelerating tissue repair, and allows for the disintegration of hyperplasia granulation tissue and the recombination of collagen fiber, and thereby reducing or avoiding the formation of scars. The eye drops of the deproteinized calf blood extract has a good surface activity and good eye tolerance, can distribute uniformly on the surface of cornea and conjunctiva so as to lubricate cornea and reduce the mechanical friction of eyelid on the cornea surface, and reduce the symptoms of topical irritation. Hence, as for corneal diseases especially corneal injury, the use of the eye drops of the deproteinized calf blood extract in combination with a conventional treatment as basis can promote the growth and repair of corneal epithelium and accelerate injure healing.

The eye drops of the deproteinized calf blood extract as proved by the present invention can be used for the treatment of corneal and conjunctival disorders, as well as xerophthalmia, thereby providing a new choice for the treatment of traumatic, inflammatory and trophic disorders of cornea and conjunctiva and xerophthalmia.

CONCRETE MODES FOR CARRYING OUT THE INVENTION

The present invention is further illustrated by the following specific examples, but it should be understood that these examples are merely used to illustrate in details, and not intended to limit the present invention in any way.

The materials and experimental methods used herein are generally and/or specifically described. Although many materials and procedural methods used for carrying out the present invention are well known in the art, the present invention still provide description in details as much as possible. It is clear for the person skilled in the art that in the context, the materials and procedural methods used in the present invention are well known in the art, unless specified otherwise.

The total solid content of the deproteinized calf blood extract used in the examples was not less than 35mg/ml, unless specified otherwise.

Example 1

Formula:

| | |
|---|---|
| Deproteinized calf blood extract | 200ml |
| Phenoxyethanol | 5.0g |
| benzalkonium chloride | 0.1g |
| Chitosan oligosaccharide | 0.5g |
| Water for injection | q.s. |
| Total volume | 1000ml |

Process of preparation:

Under aseptic conditions, phenoxyethanol, benzalkonium chloride and chitosan oligosaccharide in the prescribed amounts were respectively weighed, and dissolved in a suitable amount of water for injection with heating. The solution was cooled to room temperature and added with the deproteinized calf blood extract and water for injection to reach the total amount. It was firstly filtered by using 0.45 $\mu$m microporous membrane, then filtered to remove bacteria by using 0.22 $\mu$m microporous membrane, and filled to obtain the eye drops. The chitosan oligosaccharide used in the example had a molecular weight of not greater than 5000.

Example 2

Formula:

| | |
|---|---|
| Deproteinized calf blood extract | 200ml |
| Phenoxyethanol | 5.0g |
| Ethyl p-hydroxybenzoate | 0.1g |
| Chitosan oligosaccharide | 0.5g |
| Water for injection | q.s. |
| Total volume | 1000ml |

Process of preparation:

Under aseptic conditions, phenoxyethanol, ethyl p-hydroxybenzoate and chitosan oligosaccharide in the prescribed amounts were respectively weighed, and dissolved in a suitable amount of water for injection with heating. The solution was cooled to room temperature and added with the deproteinized calf blood extract and water for injection to reach the total amount. It was firstly filtered by using 0.45 $\mu$m microporous membrane, then filtered to remove bacteria by using 0.22 $\mu$m microporous membrane, and filled to obtain the eye drops. The chitosan oligosaccharide used in the example had a molecular weight of not greater than 3000.

Example 3

Formula:

| Deproteinized calf blood extract | 200ml |
|---|---|
| Trichloro-tert-butanol | 1.0g |
| Benzalkonium chloride | 0.1g |
| Chitosan oligosaccharide | 1.0g |
| Water for injection | q.s. |
| Total volume | 1000ml |

Process of preparation:

Under aseptic conditions, trichloro-tert-butanol, benzalkonium chloride and chitosan oligosaccharide in the prescribed amounts were respectively weighed, and dissolved in a suitable amount of water for injection with heating. The solution was cooled to room temperature and added with the deproteinized calf blood extract and water for injection to reach the total amount. It was firstly filtered by using 0.45 $\mu$m microporous membrane, then filtered to remove bacteria by using 0.22 $\mu$m microporous membrane, and filled to obtain the eye drops. The chitosan oligosaccharide used in the example had a molecular weight of not greater than 1500.

Example 4

Formula:

| Deproteinized calf blood extract | 200ml |
|---|---|
| Trichloro-tert-butanol | 1.0g |
| Ethyl p-hydroxybenzoate | 0.1g |
| Chitosan oligosaccharide | 1.0g |
| Water for injection | q.s. |
| Total volume | 1000ml |

Process of preparation:

Under aseptic conditions, trichloro-tert-butanol, ethyl p-hydroxybenzoate and chitosan oligosaccharide in the prescribed amounts were respectively weighed, and dissolved in a suitable amount of water for injection with heating. The solution was cooled to room temperature and added with the deproteinized calf blood extract and water for injection to reach the total amount. It was firstly filtered by using 0.45 $\mu$m microporous membrane, then filtered to remove bacteria by using 0.22 $\mu$m microporous membrane, and filled to obtain the eye drops. The chitosan oligosaccharide used in the example had a molecular weight of not greater than 1500.

Comparative Example 1

Formula:

| Deproteinized calf blood extract | 200ml |
|---|---|
| Trichloro-tert-butanol | 1.0g |
| Benzalkonium chloride | 0.1g |
| Water for injection | q.s. |

(continued)

| Total volume | 1000ml |
|---|---|

Process of preparation: except for adding chitosan oligosaccharide, other procedures were the same as those of Example 3, and it was filled to obtain the eye drops.

Comparative Example 2

Formula:

| Deproteinized calf blood extract | 200ml |
|---|---|
| Trichloro-tert-butanol | 1.0g |
| Ethyl p-hydroxybenzoate | 0.1g |
| Water for injection | q.s. |
| Total volume | 1000ml |

Process of preparation: except for adding chitosan oligosaccharide, other procedures were the same as those of Example 4, and it was filled to obtain the eye drops.

Test of irritation on eye

(1) Experimental materials

Tested drugs: the eye drops of Example 1 of the present application, and the blank control of eye drops provided by the applicant.
Animals: rabbits, both male and female, Japanese big-ear white rabbits, weighing 2.0-3.0 kg, provided by the Center of Experimental Animals of Jinzhou Medical College.

(2) Experimental methods

Observation of irritation on rabbit eyes after multiple dosing: 6 healthy rabbits were used, examined on both eyes within 24 hrs before the test, and the rabbits without symptoms of eye irritation, corneal defects and corneal injury after examination were used in the test, including both male and female rabbits. The ophthalmic liquid preparation of the deproteinized calf blood extract was applied to one side of conjunctival sac of each eye, while the blank solution as a control was applied to the other side, dropping amount of 0.1 ml, 4 times per day for consecutive 7 days, and allowing eyelids passively closed for about 10 s. The topical reactions were observed every day before administration and at 6, 24, 48, 72 h after the last administration. The results in comparison with the control eye are shown in Table 1.

Table 1: Eye irritation test results of multiple dosing (X$\pm$SD)(n=4)

| | | Score of irritation at varioius time points | |
|---|---|---|---|
| | | Drug group | Blank control group |
| duration of administration (day) | 1 | 0 | 0 |
| | 2 | 0 | 0 |
| | 3 | 0 | 0 |
| | 4 | 0 | 0 |
| | 5 | 0 | 0 |
| | 6 | 0 | 0 |
| | 7 | 0 | 0 |

(continued)

|  |  | Score of irritation at varioius time points | |
| --- | --- | --- | --- |
|  |  | Drug group | Blank control group |
| After the last administration (hour) | 6 | 0 | 0 |
|  | 24 | 0 | 0 |
|  | 48 | 0 | 0 |
|  | 72 | 0 | 0 |

The results in the table show that the drug group and the control group did not produce abnormality during the period of administration and at 6, 24, 48 and 74 h after the last administration with a score of 0, indicating that the eye drops has no irritation.

(3) Conclusion: the eye drops of the deproteinized calf blood extract after multiple dosing has no irritation on eye.

Allergy test on topical skins

(1) Experimental materials

Tested drugs: the eye drops of Example 1 of the present application, and blank control of the eye drops, positive sensitizing agent (2,4-dinitrochlorobenzene) provided by the applicant.

Animals: white guinea pigs, both male and female, weighing 250 - 300 g, provided by the Center of Experimental Animals of Jinzhou Medical College.

(2) Experimental methods

30 white guinea pigs both male and female were used, randomly divided into 3 groups, 10 for each group. The both sides of the back of the guinea pigs were epilated at 24h before administration. The eye drops of the present application was applied to Group I; the blank control was applied to Group II; and the positive sensitizing agent was applied to Group III.

Sensitizing contact: the ophthalmic liquid preparation of 0.2 ml was applied on the left epilated area of the back of the guinea pigs, kept for 6 h, and the same procedure was repeated once on the 7th and 14th day, respectively. The blank control group and the positive sensitizing agent group were treated by the same procedures as those of the ophthalmic liquid preparation group.

Stimulating contact: at day 14 after the last administration of the ophthalmic liquid preparation, the ophthalmic liquid preparation of 0.2 ml was applied on the right epilated area of the back of the guinea pigs, the ophthalmic liquid preparation was removed after 6h, observation was conducted immediately, and then the skin sensitization was observed again at 24, 48 and 72 h. Scoring was performed according to the scoring standard of conventional safety tests of preparations prescribed in "Preclinical safety evaluation and practice of new drugs", the scoring standard for skin sensitization was shown in Table 2, and the categories of sensitization rate were given in Table 3. The blank control group and the positive sensitizing agent group were treated by the same procedures as those of the ophthalmic liquid preparation group.

Table 2: Scoring standard for skin sensitization

| Skin sensitization | Score |
| --- | --- |
| Formation of red spots | |
| no red spots | 0 |
| mild red spots | 1 |
| moderate red spots | 2 |
| sever red spots | 3 |
| edematous red spots | 4 |

(continued)

| Edematization | |
|---|---|
| no edematization | 0 |
| mild edematization | 1 |
| moderate edematization | 2 |
| sever edematization | 3 |
| total scores | 7 |

$$\text{Average value of sensitization} = \frac{\text{Total score of red spot formation} + \text{Total score of edematization}}{\text{Total animal number}}$$

Table 3: Categories of sensitization rate

| Sensitization rate (%) | Sensitization intensity |
|---|---|
| 0~10 | weak sensitization |
| 20~30 | mild sensitization |
| 40~60 | moderate sensitization |
| 70~80 | high sensitization |
| 90~100 | extremely high sensitization |

Results: Results of skin sensitization scoring were given in Table 4.

Table 4: Results of topical skin sensitization test of the eye drops of the deproteinized calf blood extract

| Group | | 6h | 24h | 48h | 72h |
|---|---|---|---|---|---|
| Group I | red spot | 0 | 0 | 0 | 0 |
| | edema | 0 | 0 | 0 | 0 |
| | score | 0 | 0 | 0 | 0 |
| Group II | red spot | 0 | 0 | 0 | 0 |
| | edema | 0 | 0 | 0 | 0 |
| | score | 0 | 0 | 0 | 0 |
| Group III | red spot | 3.5±0.53 | 3.8±0.42 | 3.6±0.52 | 3.3±0.48 |
| | edema | 2.8±0.42 | 2.9±0.32 | 2.8±0.42 | 2.7±0.48 |
| | score | 6.3±0.48 | 6.7±0.67 | 6.4±0.70 | 5.8±0.42 |

The eye drops of the deproteinized calf blood extract was applied on the skin of the guinea pigs for sensitizing contact and stimulating contact, did not result in red spots and edema, and showed no difference from the blank control group, while the positive sensitizing agent group gave a sensitization rate of 100%, which indicated that the present eye drops has no topical skin sensitization.

(3) Conclusion:

The ophthalmic liquid preparation of the deproteinized calf blood extract showed no topic sensitization after administration in guinea pigs.

Stability investigation

(1) Experimental materials

Tested drugs: the eye drops of Examples 3 and 4 of the present application and the eye drops of Comparative Examples 1 and 2 (referred to as Control 1 and Control 2 hereafter, respectively) provided by the applicant.

(2) Investigated items

Measurement of pH value: conducted by referring to the Section H, Supplement VI, Second Part, the Chinese Pharmacopoeia, 2005. The pH values were between 6.0 and 9.0.

Visible foreign matters: examined by referring to the Section H "Methods for examining visible foreign matters", Supplement IX, Second Part, the Chinese Pharmacopoeia, 2005.

Vitality test: the drugs were sampled, measured for the respiration vitality $QO_2$ of liver homogenates from guinea pigs by a Warburg manometer, and the respiration vitality should be no less than 4.0 $\mu$l $O_2$/mg·h.

Polypeptide content: measured using a spectrophotometry by referring to section 16-119 of the Folin-Phenol method, Vol. 16, the National Drug Standards,.

(3) Experimental methods

The eye drops was sampled, stood at a temperature of 25°C±2°C and a relative humidity of 60%±5%, and tested for the observed items at 0 month, 3 months, 6 months, 9 months, 12 months, 16 months, 18 months, 20 months, 22 months and 24 months, compared with the sample at 0 month. The results are shown in Tables 5 and 6.

Table 5: Results of stability investigation (1)

| Item Time | | 0 month | 3 months | 6 month | 9 month | 12 month |
|---|---|---|---|---|---|---|
| pH value | Example 3 | 6.80 | 6.82 | 6.87 | 6.88 | 6.86 |
| | Example 4 | 6.82 | 6.85 | 6.89 | 6.91 | 6.92 |
| | Control 1 | 6.84 | 6.86 | 6.88 | 6.85 | 6.87 |
| | Control 2 | 6.80 | 6.82 | 6.89 | 6.88 | 6.94 |
| Visible foreign matters | Example 3 | clear | clear | clear | clear | clear |
| | Example 4 | clear | clear | clear | clear | clear |
| | Control 1 | clear | clear | clear | clear | clear |
| | Control 2 | clear | clear | clear | clear | clear |
| Vitality test $QO_2$ ($\mu$l/mg·h) | Example 3 $QO_2$ | 6.98 | 6.88 | 6.62 | 6.46 | 6.12 |
| | Example 4 $QO_2$ | 6.95 | 6.87 | 6.60 | 6.44 | 6.15 |
| | Control 1 $QO_2$ | 6.97 | 6.76 | 6.37 | 5.69 | 4.84 |
| | Control 2 $QO_2$ | 6.98 | 6.79 | 6.31 | 5.61 | 4.80 |
| polypeptide content | Example 3 | 0.237 | 0.237 | 0.239 | 0.238 | 0.236 |
| | Example 4 | 0.24 | 0.241 | 0.238 | 0.238 | 0.237 |
| | Control 1 | 0.238 | 0.237 | 0.238 | 0.236 | 0.236 |
| | Control 2 | 0.236 | 0.238 | 0.237 | 0.236 | 0.236 |

Table 6: Results of stability investigation (2)

| Item Time | | 16 month | 18 month | 20 month | 22 month | 24 month |
|---|---|---|---|---|---|---|
| pH value | Example 3 | 6.88 | 6.86 | 6.83 | 6.82 | 6.94 |
| | Example 4 | 6.91 | 6.86 | 6.93 | 6.88 | 6.88 |
| | Control 1 | 6.88 | 6.82 | 6.88 | 6.92 | 6.94 |
| | Control 2 | 6.92 | 6.88 | 6.84 | 6.87 | 6.91 |
| Visible foreign matters | Example 3 | clear | clear | clear | clear | clear |
| | Example 4 | clear | clear | clear | clear | clear |
| | Control 1 | clear | clear | clear | clear | clear |
| | Control 2 | clear | clear | clear | clear | clear |
| Vitality test $QO_2$ ($\mu$l)/mg·h) | Example 3 $QO_2$ | 5.79 | 5.54 | 5.32 | 5.04 | 4.73 |
| | Example 4 $QO_2$ | 5.77 | 5.51 | 5.28 | 5.00 | 4.68 |
| | Control 1 $QO_2$ | 4.36 | 4.08 | 3.86 | 3.47 | 3.11 |
| | Control 2 $QO_2$ | 4.38 | 4.05 | 3.81 | 3.44 | 3.08 |
| Polypeptide content | Example 3 | 0.237 | 0.239 | 0.235 | 0.231 | 0.227 |
| | Example 4 | 0.238 | 0.239 | 0.236 | 0.228 | 0.218 |
| | Control 1 | 0.237 | 0.235 | 0.231 | 0.216 | 0.198 |
| | Control 2 | 0.237 | 0.236 | 0.233 | 0.219 | 0.207 |

According to the requirements for the investigation, the vitality should meet the requirement of $QO_2 \geq 4$. From the results, it can be seen that in comparison with the drops without using chitosan oligosaccharide, the present eye drops still met the requirements of protein vitality after 18 months, which indicated that chitosan oligosaccharide could protect protein respiration vitality to some extent to further stabilize the preparation. Hence, the results of stability investigation confirmed that the addition of the chitosan oligosaccharide significantly improved the stability of the eye drops of the present invention.

**Claims**

1. Eye drops comprising a therapeutically effective amount of a deproteinized calf blood extract, chitosan oligosaccharide, and water.

2. The eye drops of claim 1 comprising 10-30% (v/v) of the deproteinized calf blood extract, 0.01-1% (w/v) of the chitosan oligosaccharide, and water, based on the total amount of the eye drops.

3. The eye drops of claim 2 wherein the chitosan oligosaccharide has a molecular weight of less than 5000, preferably less than 3000, preferably less than 2000, and preferably less than 1500.

4. The eye drops of any one of claims 1-3 which further comprises one or more bacteriostatic agents selected from the group consisting of: phenylethanol, phenoxyethanol, methyl p-hydroxybenzoate, ethyl p-hydroxybenzoate, propyl p-hydroxybenzoate, benzalkonium chloride, and trichloro-tert-butanol.

5. The eye drops of any one of claims 1-4 which further comprises a pH regulator.

6. The eye drops of any one of claims 1-5 which further comprises an osmotic regulator.

7. The eye drops of any one of claims 1-6 which further comprises a viscosity regulator.

8. The eye drops of any one of claims 1-7 which has any one of the following formulas:

i)

| | |
|---|---|
| the deproteinized calf blood extract | 200 parts by weight, |
| phenoxyethanol | 5 parts by weight, |
| benzalkonium chloride | 0.1 parts by weight, |
| chitosan oligosaccharide | 0.5 parts by weight, |
| water for injection | q.s., to 1000 parts by weight; |

ii)

| | |
|---|---|
| the deproteinized calf blood extract | 200 parts by weight, |
| phenoxyethanol | 5 parts by weight, |
| ethyl p-hydroxybenzoate | 0.1 parts by weight, |
| chitosan oligosaccharide | 0.5 parts by weight, |
| water for injection | q.s., to 1000 parts by weight: |

iii)

| | |
|---|---|
| the deproteinized calf blood extract | 200 parts by weight, |
| trichloro-tert-butanol | 1 parts by weight, |
| benzalkonium chloride | 0.1 parts by weight, |
| chitosan oligosaccharide | 1.0 part by weight, |
| water for injection | q.s.. to 1000 parts bv weight; |

or
iv)

| | |
|---|---|
| the deproteinized calf blood extract | 200 parts by weight, |
| trichloro-tert-butanol | 1.0 part by weight, |
| ethyl p-hydroxybenzoate | 0.1 parts by weight, |
| chitosan oligosaccharide | 1.0 part by weight, |
| water for injection | q.s.. to 1000 parts bv weight. |

9. Use of the eye drops of any one of claims 1-8 in manufacture of a medicament for the treatment of an ophthalmic disease.

10. Use according to claim 9, wherein the ophthalmic disease is selected from the group consisting of a keratopathy, such as a corneal injury, a pathology of conjunctiva, and xerophthalm ia.

11. The eye drops of any one of claims 1-8 for use as a medicament.

12. The eye drops of any one of claims 1-8 for use in the treatment and/or prophylaxis of an ophthalm ic disease

13. The eye drops according to claim 12, wherein the ophthalmic disease is selected from the group consisting of a keratopathy, such as a corneal injury, a pathology of conjunctiva, and xerophthalmia.

**Patentansprüche**

1. Augentropfen, welche eine therapeutisch effektive Menge eines deproteinisierten Kälberblut-Extraktes, Chitosan-Oligosaccharid und Wasser umfassen.

2. Augentropfen nach Anspruch 1, welche 10 - 30 % (Vol./Vol.) des deproteinisierten Kälberblut-Extraktes, 0,01 - 1 % (Gew./Vol.) des Chitosan-Oligosaccharides und Wasser, auf der Grundlage der Gesamtmenge der Augentropfen, umfassen.

3. Augentropfen nach Anspruch 2, wobei das Chitosan-Oligosaccharid ein Molekulargewicht von weniger als 5.000, vorzugsweise weniger als 3.000, vorzugsweise weniger als 2.000, und vorzugsweise weniger als 1.500 aufweist.

4. Augentropfen nach einem der Ansprüche 1 bis 3, welche ferner ein oder mehrere bakteriostatische Mittel ausgewählt aus der Gruppe bestehend aus: Phenylethanol, Phenoxyethanol, Methyl-p-Hydroxybenzoat, Ethyl-p-Hydroxybenzoat, Propyl-p-Hydroxybenzoat, Benzalkoniumchlorid und Trichlor-tert-Butanol umfassen.

5. Augentropfen nach einem der Ansprüche 1 bis 4, welche ferner einen pH-Regulator umfassen.

6. Augentropfen nach einem der Ansprüche 1 bis 5, welche ferner einen osmotischen Regulator umfassen.

7. Augentropfen nach einem der Ansprüche 1 bis 6, welche ferner einen Viskositätsregulator umfassen.

8. Augentropfen nach einem der Ansprüche 1 bis 7, welche eine der folgenden Formeln aufweisen:

   i)

   | | |
   |---|---|
   | das deproteinisierte Kälberblut-Extrakt | 200 Gewichtsteile |
   | Phenoxyethanol | 5 Gewichtsteile |
   | Benzalkoniumchlorid | 0,1 Gewichtsteile |
   | Chitosan-Oligosaccharid | 0,5 Gewichtsteile |
   | Wasser zur Injektion | q.s., auf 1.000 Gewichtsteile |

   ii)

   | | |
   |---|---|
   | das deproteinisierte Kälberblut-Extrakt | 200 Gewichtsteile |
   | Phenoxyethanol | 5 Gewichtsteile |
   | Ethyl-p-Hydroxybenzoat | 0,1 Gewichtsteile |
   | Chitosan-Oligosaccharid | 0,5 Gewichtsteile |
   | Wasser zur Injektion | q.s., auf 1.000 Gewichtsteile |

   iii)

   | | |
   |---|---|
   | das deproteinisierte Kälberblut-Extrakt | 200 Gewichtsteile |
   | Trichlor-tert-Butanol | 1 Gewichtsteil |
   | Benzalkoniumchlorid | 0,1 Gewichtsteile |
   | Chitosan-Oligosaccharid | 1,0 Gewichtsteil |
   | Wasser zur Injektion | q.s., auf 1.000 Gewichtsteile |

   oder
   iv)

   | | |
   |---|---|
   | das deproteinisierte Kälberblut-Extrakt | 200 Gewichtsteile |
   | Trichlor-tert-Butanol | 1,0 Gewichtsteil |
   | Ethyl-p-Hydroxybenzoat | 0,1 Gewichtsteile |
   | Chitosan-Oligosaccharid | 1,0 Gewichtsteil |
   | Wasser zur Injektion | q.s., auf 1.000 Gewichtsteile |

9. Verwendung der Augentropfen nach einem der Ansprüche 1 bis 8 bei der Herstellung eines Medikamentes für die Behandlung einer Augenerkrankung.

10. Verwendung nach Anspruch 9, wobei die Augenerkrankung ausgewählt ist aus der Gruppe bestehend aus einer Keratopathie, wie z.B. eine Hornhautverletzung, einer Pathologie der Bindehaut und Xerophthalmie.

11. Augentropfen nach einem der Ansprüche 1 bis 8 zur Verwendung als ein Medikament.

12. Augentropfen nach einem der Ansprüche 1 bis 8 zur Verwendung bei der Behandlung und/oder Prophylaxe einer Augenerkrankung.

13. Augentropfen nach Anspruch 12, wobei die Augenerkrankung ausgewählt ist aus der Gruppe bestehend aus einer Keratopathie, wie z.B. eine Hornhautverletzung, einer Pathologie der Bindehaut und Xerophthalmie.

**Revendications**

1. Gouttes ophtalmiques comprenant une quantité thérapeutiquement efficace d'un extrait de sang de veau déprotéinisé, d'oligosaccharide de chitosane, et d'eau.

2. Gouttes ophtalmiques selon la revendication 1, comprenant de 10 à 30 % (volume / volume) de l'extrait de sang de veau déprotéinisé, 0,01 à 1 % (poids / volume) de l'oligosaccharide de chitosane, et de l'eau, sur la base de la quantité totale des gouttes ophtalmiques.

3. Gouttes ophtalmiques selon la revendication 2, dans lesquelles l'oligosaccharide de chitosane possède un poids moléculaire inférieur à 5 000, de préférence inférieur à 3 000, préférablement inférieur à 2 000, et préférablement inférieur à 1 500.

4. Gouttes ophtalmiques selon l'une quelconque des revendications 1 à 3, qui comprennent en outre un ou plusieurs agents bactériostatiques sélectionnés parmi le groupe constitué de : phényléthanol, phénoxyéthanol, méthyle p-hydroxybenzoate, éthyle p-hydroxybenzoate, propyle p-hydroxybenzoate, chlorure de benzalkonium, et trichloro-tert-butanol.

5. Gouttes ophtalmiques selon l'une quelconque des revendications 1 à 4, qui comprennent en outre un régulateur de pH.

6. Gouttes ophtalmiques selon l'une quelconque des revendications 1 à 5, qui comprennent en outre un régulateur osmotique.

7. Gouttes ophtalmiques selon l'une quelconque des revendications 1 à 6, qui comprennent en outre un régulateur de viscosité.

8. Gouttes ophtalmiques selon l'une quelconque des revendications 1 à 7, qui possèdent l'une quelconque des formules suivantes :

   i)

   | | |
   |---|---|
   | l'extrait de sang de veau déprotéinisé | 200 parties en poids, |
   | phénoxyéthanol | 5 parties en poids, |
   | chlorure de benzalkonium | 0,1 partie en poids, |
   | oligosaccharide de chitosane | 0,5 partie en poids, |
   | eau pour injection | q.s.p. pour 1 000 parties en poids ; |

   ii)

   | | |
   |---|---|
   | l'extrait de sang de veau déprotéinisé | 200 parties en poids, |
   | phénoxyéthanol | 5 parties en poids, |

(suite)

| | |
|---|---|
| éthyle p-hydroxybenzoate | 0,1 partie en poids, |
| oligosaccharide de chitosane | 0,5 partie en poids, |
| eau pour injection | q.s.p. pour 1 000 parties en poids ; |

iii)

| | |
|---|---|
| l'extrait de sang de veau déprotéinisé | 200 parties en poids, |
| trichloro-tert-butanol | 1 partie en poids, |
| chlorure de benzalkonium | 0,1 partie en poids, |
| oligosaccharide de chitosane | 1,0 partie en poids, |
| eau pour injection | q.s.p. pour 1 000 parties en poids ; |

ou
iv)

| | |
|---|---|
| l'extrait de sang de veau déprotéinisé | 200 parties en poids, |
| trichloro-tert-butanol | 1,0 partie en poids, |
| éthyle p-hydroxybenzoate | 0,1 partie en poids, |
| oligosaccharide de chitosane | 1,0 partie en poids, |
| eau pour injection | q.s.p. pour 1 000 parties en poids. |

9. Utilisation des gouttes ophtalmiques selon l'une quelconque des revendications 1 à 8 dans la fabrication d'un médicament pour le traitement d'une maladie ophtalmique.

10. Utilisation selon la revendication 9, dans laquelle la maladie ophtalmique est sélectionnée parmi le groupe constitué d'une kératopathie, telle qu'une lésion cornéenne, une pathologie de conjonctive, et une xérophtalmie.

11. Gouttes ophtalmiques selon l'une quelconque des revendications 1 à 8, à utiliser comme médicament.

12. Gouttes ophtalmiques selon l'une quelconque des revendications 1 à 8, à utiliser dans le traitement et / ou la prophylaxie d'une maladie ophtalmique.

13. Gouttes ophtalmiques selon la revendication 12, dans laquelle la maladie ophtalmique est sélectionnée parmi le groupe constitué d'une kératopathie, telle qu'une lésion cornéenne, une pathologie de conjonctive, et une xérophtalmie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 1895275

**Non-patent literature cited in the description**

• *Section H, Supplement VI, Second Part, the Chinese Pharmacopoeia,* 2005
• Methods for examining visible foreign matters. *Supplement IX, Second Part, the Chinese Pharmacopoeia,* 2005
• Folin-Phenol method. National Drug Standards, vol. 16